# FASCICULE DE BREVET EUROPEEN

(11) **EP 1 303 759 B1**
(45) Date de publication et mention de la délivrance du brevet: **12.10.2005**
(21) Numéro de dépôt: 01947607.6
(22) Date de dépôt: 25.06.2001
(51) Int. Cl.: G01N 33/58

(54) **IMMUNODOSAGES ELECTROCHIMIQUES A L'AIDE DE MARQUEURS METALLIQUES COLLOIDAUX**
ELEKTROCHEMISCHER IMMUNOASSAY MIT KOLLOIDALEM METALL
ELECTROCHEMICAL IMMUNOASSAYS USING COLLOIDAL METAL MARKERS

(30) Priorité: 26.06.2000 FR 0008145
(43) Date de publication de la demande: 23.04.2003
(73) Titulaire: CENTRE NATIONAL DE LA RECHERCHE SCIENTIFIQUE (CNRS), 75794 Paris Cedex 16 (FR)
(72) Inventeur: LIMOGES, Benoît, F-63360 Gerzat (FR); AUTHIER, Laurent, F-63170 Aubiere (FR); DEQUAIRE, Murielle, F-63000 Clermont-Ferrand (FR)
(74) Mandataire: Warcoin, Jacques
(86) Numéro de dépôt international: PCT/FR2001/002000
(87) Numéro de publication internationale: WO 2002/001178

(56) Documents cités:
- EP-A- 0 310 872
- WO-A-97/04313
- WO-A-99/32662
- US-A- 5 637 508
- GONZALEZ GARCIA M B ET AL: "Adsorptive stripping voltammetric behaviour of colloidal gold and immunogold on carbon paste electrode." BIOELECTROCHEMISTRY AND BIOENERGETICS, vol. 38, no. 2, 1995, pages 389-395, XP000609801 ISSN: 0302-4598 cité dans la demande
- MCCARTHY, A. J. ET AL: "The mechanism of the oxidative dissolution of colloidal gold in cyanide media" J. ELECTROCHEM. SOC. (1998), 145(2), 408-414, XP000993453
- DEQUAIRE, MURIELLE ET AL: "An Electrochemical Metalloimmunoassay Based on a Colloidal Gold Label" ANAL. CHEM. (2000), 72(22), 5521-5528, XP000996878
- GONZALEZ-GARCIA M B ET AL: "Colloidal gold as an electrochemical label of streptavidin-biotin interaction." BIOSENSORS & BIOELECTRONICS, vol. 15, no. 5-6, août 2000 (2000-08), pages 315-321, XP001001496 ISSN: 0956-5663

## Description

La détection ou la quantification de substances biologiques à partir de méthodes d'immunodosage ou encore de dosage de fragments d'ADN par hybridation d'acides nucléiques, sont extrêmement importantes dans de nombreux domaines de la biologie clinique (recherche médicale et biologique, diagnostic, génétique, dépistage de substances illicites à l'état de traces, etc.) ou même dans le domaine de l'environnement (détection de contaminants comme les pesticides ou les bactéries). Ces méthodes permettent de répondre au double critère de sélectivité et sensibilité. Parmi elles, l'immunoanalyse avec marqueur, basée sur une reconnaissance affine antigène/anticorps, est particulièrement performante et elle s'est répandue grâce au développement d'une large gamme de marqueurs non radioactifs tels que les marqueurs enzymatiques, fluorescents ou chimioluminescents, couplés d'une manière générale à une détection spectroscopique. Cependant, chaque marqueur a ses propres avantages et inconvénients. En effet un marqueur idéal doit répondre à diverses exigences ; il doit :
1) être détectable de façon sensible par des instruments analytiques de faible coût et de manipulation aisée,
2) permettre à la molécule marquée (traceur) de rester soluble et stable dans le milieux du dosage,
3) autoriser un marquage simple et efficace, à un prix raisonnable,
4) être de durée de vie élevée,
5) être sans risque pour la santé du manipulateur,
6) conduire à un traceur ayant une réactivité proche de la molécule non marquée,
7) conduire à un bruit de fond minimal.

Parmi les marqueurs commercialisés, les marqueurs fluorescents et luminescents, développés au début des années 70, présentent de nombreux avantages : ils sont en général non toxiques et stables, et leur détection est très sensible. Cependant, ils nécessitent un appareillage relativement sophistiqué et onéreux, et la mesure est souvent affectée par une fluorescence endogène liée à des effets de matrices de l'échantillon.

Les marqueurs enzymatiques apparus en même temps que les marqueurs fluorescents, sont probablement aujourd'hui les plus populaires en raison de leur propriété catalytique remarquable, mais également par leur faculté de déclencher, pour certains substrats, des réactions colorées qui autorisent l'emploi d'un détecteur très simple comme un colorimètre, voire même l'oeil d'un manipulateur. Les marqueurs enzymatiques sont à la base des méthodes appelées ELISA (Enzyme-Linked ImmunoSorbant Assay). Ils ont cependant, eux aussi, leurs propres inconvénients. Certaines substances présentes dans l'échantillon peuvent inhiber l'enzyme. En outre, ils sont relativement fragiles et de durée de vie limitée. Par ailleurs, le bruit de fond peut être important.

Les marqueurs à base de métaux ont été introduits vers la fin des années 70, avec en partie pour but de remédier à certains des inconvénients cités précédemment. Les marqueurs à base de métaux se distinguent selon leur nature chimique, à savoir, les particules métalliques colloïdales, les ions métalliques, les complexes de coordination, les organométalliques ou encore les métallo-protéines. En fonction de leur nature, différentes techniques analytiques peuvent leur être associées comme la fluorescence résolue dans le temps, la spectrophotométrie d'absorption atomique, l'infrarouge à transformée de Fourier, ou encore les techniques électrochimiques telles que la polarographie ou la voltammétrie.

Comparées aux méthodes spectrophotométriques, les techniques électrochimiques présentent de nombreux avantages : les mesures peuvent être effectuées dans de très faibles volumes de liquide (inférieurs au microlitre), en milieu éventuellement trouble (cas des sérums), avec la possibilité d'offrir une bonne sensibilité pour un appareillage de faible coût, éventuellement portable (de taille réduite). Bien que les techniques électrochimiques permettent de détecter des marqueurs organométalliques ou des métaux ioniques jusqu'à des concentrations nanomolaires (10⁻⁹ M), cela reste cependant souvent insuffisant comparé aux marqueurs fluorescents qui peuvent, quant à eux, être détectés jusqu'à des seuils picomolaires (10⁻¹² M). La stratégie de détection électrochimique développée dans la présente invention montre qu'il est possible d'atteindre des concentrations en un marqueur métallique de l'ordre de 10⁻¹² M.

L'invention concerne plus précisément un procédé de détection électrochimique d'une particule métallique colloïdale utilisée comme marqueur dans un immunodosage. L'invention concerne également la détermination quantitative ou qualitative de composés pouvant être des haptènes, antigènes, anticorps, mais également des composés tels que des fragments d'ADN ou d'ARN. De manière générale, l'invention peut être étendue à toutes méthodes d'analyse faisant intervenir une interaction spécifique et affine entre un ligand et une molécule hôte, et dans laquelle il est nécessaire d'ajouter un marqueur permettant de quantifier sensiblement ladite interaction. D'autre part, de nombreux formats d'immunodosages, qu'ils soient compétitifs ou non compétitifs, ou de procédés d'hybridation d'acides nucléiques, de préférence en phase hétérogène, peuvent être appliqués.

L'utilisation de particules métalliques colloïdales comme marqueur n'est pas nouvelle. En effet, celles-ci sont très couramment employées comme agent de contraste dans des techniques de microscopie électronique, notamment sous forme de colloïdes d'or couplés à des anticorps dans le but, par exemple, de déterminer la distribution d'un antigène à la surface d'une cellule (Beesley, *Proceedings RMS*, **1985,** 20, 187-196). En revanche l'emploi d'une particule métallique colloïdale comme marqueur dans le contexte d'un dosage par affinité est peu commun. A ce titre, on peut noter l'existence d'un brevet (US 4313734) et d'une publication (Leuvering *et al., J. Immunoassay* **1980,** 1, 71-91), relatant l'utilisation d'un marqueur à base d'or ou d'argent colloïdal dans le contexte d'un immunodosage avec détection par absorption atomique ou colorimétrique. D'autres brevets assez similaires relatent également l'emploi de colloïde d'or comme marqueurs dans des immunodosages avec détection colorimétrique (US 4853335, EP0310872, EP0258963). Une détection également colorimétrique a été récemment décrite pour l'analyse de fragments d'ADN par hybridation *via* un marqueur d'or colloïdal *(Storhoff et al*., *J. Am. Chem. Soc.* **1998,** 120, 1959-1964).

En ce qui concerne une détection électrochimique, il ne semble pas qu'une méthode d'immunoanalyse ou de dosage d'ADN par hybridation impliquant la détection ou la quantification électrochimique d'un marqueur métallique colloïdal n'ait pour l'instant été décrite. On peut cependant relever l'existence d'un article concernant la détection directe d'un colloïde d'or recouvert d'anticorps et adsorbé à la surface d'une électrode à pâte de carbone (Gonzalez-Garcia et Costa-Garcia, *Bioelectrochem. Bioenerg*. **1995,** 38, 389-395). Cependant l'application à un immunodosage, bien qu'envisagée, n'a pas été démontrée. WO 9704313 décrit l'application à un immunodosage de cette méthode sans donner des résultats expérimentaux.

Les Inventeurs ont cherché à vérifier s'il était oui ou non possible de réaliser un immunodosage tel que l'envisageaient les auteurs, c'est-à-dire un immunodosage procédant à la surface même de l'électrode et pour lequel, après immunoréaction, le marqueur d'or colloïdal ayant réagit à proximité de la surface de l'électrode est directement détecté. Le résultat de cette expérience a permit de conclure qu'il n'était pas possible de détecter le colloïde d'or de cette manière, probablement parce que ce dernier n'est plus en contact immédiat avec la surface de l'électrode. La présente invention permet de s'affranchir de ce problème grâce à une détection indirecte du marqueur métallique colloïdal, autorisant par la même l'utilisation d'une phase solide pouvant être autre que la surface de l'électrode.

Ainsi, la présente invention permet la détection ou la quantification d'une substance biologique couplée à une particule métallique colloïdale par détection électrochimique, ladite particule métallique colloïdale étant dissoute, et détectée par électrochimie après avoir été reprécipitée à la surface de l'électrode. Ceci permet d'augmenter la concentration locale, et le seuil de sensibilité. L'objet de l'invention est donc un procédé de détection ou de quantification d'une substance biologique couplée à une particule métallique colloïdale par détection électrochimique, caractérisé en ce qu'il comprend une étape de dissolution de ladite particule métallique colloïdale.

La méthode d'immunodosage électrochimique développée dans la présente invention, peut non seulement être plus sensible que les techniques d'immunodosages enzymatiques actuelles, mais également offrir la possibilité de déterminer et/ou quantifier simultanément plusieurs composés si on utilise plusieurs marqueurs métalliques colloïdaux de nature différente. En effet, les méthodes électrochimiques autorisent la détection simultanée de plusieurs métaux au cours d'une même mesure.

D'autre part, les marqueurs métalliques colloïdaux offrent l'avantage d'être beaucoup plus stables que les marqueurs radio-isotopiques ou enzymatiques, et ils autorisent un marquage simple pour un faible coût de nombreuses substances, sans perte d'activité pour ces dernières.

Le traitement chimique permettant de dissoudre la particule métallique colloïdale s'effectue dans un milieux acide contenant un agent d'oxydation. La concentration en oxydant est choisie de manière à être en excès suffisant pour dissoudre les plus fortes concentrations en marqueur métallique colloïdal. Une solution d'acide bromhydrique contenant du brome (Br₂) ou de l'acide hypobromeux (HBrO) ou un mélange des deux comme agent d'oxydation est préféré (par exemple : 10⁻⁴ M en Br₂ dans 0,1 ou 1 M HBr), en particulier lorsque l'on veut dissoudre un colloïde d'or. Une solution d'acide chlorhydrique (par exemple 0,1 M) contenant un sel de bromure (concentration ≥ à 0,1 M) et du brome peut également convenir. Selon la nature du colloïde métallique à dissoudre, d'autres milieux acides de dissolution (H₂SO₄, HClO₄, HF, ....) et réactifs d'oxydation (I₂, Cl₂, HClO, HIO, H₂O₂, HNO₃, CN⁻, Cr₂O₄⁻, MnO₄⁻, ...) peuvent être envisagés.

Après dissolution, un traitement supplémentaire peut s'avérer nécessaire pour éliminer l'excès de réactif d'oxydation tel que le brome. Pour ce faire, un excès de phénol, aniline, hydrazine, oxine ou un de leurs dérivés, ou encore de préférence un excès d'acide 3-phenoxyacetique peut être ajouté au milieu. Ce dernier est préférable en raison d'une moindre toxicité. Une concentration de 5 × 10⁻⁴ M est généralement suffisante. Le brome peut également être éliminé par dégazage.

Il peut être avantageux d'ajouter en solution un réactif capable de complexer l'ion métallique afin de favoriser sa détection. En effet, la complexation peut transformer un ion métallique non électroactif en composé électroactif détectable. D'autre part, l'ion métallique complexé, en raison d'un caractère hydrophobe plus marqué, peut s'adsorber sur l'électrode et ainsi être plus sensiblement détecté par voltammétrie par adsorption et redissolution cathodique (van den *Berg, Anal. Chim. Acta* **1991,** 250, 265-276).

Après dissolution du métal, celui-ci est réduit à la surface de l'électrode, de préférence par application d'un potentiel très négatif. Puis, on fait varier le potentiel afin de réoxyder le métal, qui passe alors en solution. L'intensité du pic voltamétrique (surface) reflète la quantité de métal déposé sur l'électrode, donc la quantité de particules colloïdales présentes initialement dans la solution. Ceci permet donc d'effectuer le dosage. Lorsque l'on utilise des particules constituées de métaux différents, la détection simultanée est possible en raison des potentiels de réoxydation distincts des différents métaux.

Ainsi, on peut déduire la présence et/ou la quantité de substance biologique couplée initialement à la particule colloïdale, en fonction de la présence et/ou quantité de métal électrodéposé à la surface de l'électrode.

Les particules métalliques colloïdales peuvent être constituées de métal comme les colloïdes d'or, argent, cuivre, platine, rhodium, palladium, iridium, nickel, fer, ou encore de composés métalliques comme par exemple des oxydes, ou halogénures, ou chalcogénures métalliques tels que Ag₂O, AgI, Bi₂O₅, Cd₃P₂, CdS, CdSe, CdTe, Co₂O₃, CrO₃, Cu₂S, HgI₂, MnO₂, PbS, PbO₂, SnO₂, TiO₂, RuO₂, ZnO, ZnS, ZnO₂, ou des hydroxydes métalliques. De manière générale, tout métal ou composé métallique pouvant être détecté électrochimiquement peut être envisagé, de préférence les métaux de transition (van den Berg, *Anal. Chim. Acta* **1991,** 250, 265-276). La pratique veut que l'on préférera l'emploi de métaux ou composés métalliques qui ne sont que très peu ou pas présents dans le milieu du dosage, et plus particulièrement ceux qui offrent les meilleures limites de détection vis-à-vis des techniques électrochimiques employées. L'invention a été démontrée en particulier pour un colloïde d'or.

Les particules colloïdales à base de métal peuvent être obtenues à partir d'un des nombreux procédés décrits dans la littérature scientifique (Hayat, Ed, *Colloidal gold: principles, methods, and applications*; Academic Press: San Diego, CA, 1991 - Mackay, et Texter, Eds, *Electrochemistry in colloids and dispersions*, VCH Publishers: New York, 1992 - *Murray et al., J. Am. Chem. Soc.* **1993,** 115, 8706-8715 - Frens, *Nat. Phys. Sci.* **1973,** 241, 20-22 -US5637508 - Weller, *Angew. Chem., Int. Ed. Engl*. **1993,** 32, 41-43 - Wang et Herron, *J. Phys. Chem*. **1991,** 95, 525-532). Selon la méthode de fabrication, les particules peuvent avoir une taille comprise entre 1 et 200 nm, avec une très faible dispersion. Dans la présente invention, des colloïdes métalliques de taille comprise entre 5 et 100 nm sont préférés. L'emploi d'une particule de taille importante est susceptible d'améliorer la sensibilité du dosage.

Selon le format et le type de dosage, la particule métallique colloïdale peut être couplée à un anticorps, un récepteur protéinique, un antigène, un haptène, une protéine, un peptide, un oligonucléotide, un fragment d'acide nucléique (en particulier ADN ou ARN). Par couplage on entend toute méthode de liaison chimique ou physique, directe ou indirecte avec la surface de la particule, telle qu'une liaison covalente ou une adsorption *via* des interactions électrostatiques, ponts hydrogène, etc. De nombreux protocoles de couplage ont été décrits (Beesley, *Proceedings RMS,* **1985,** 20, 187-196 - Oliver, *Methods in molecular biology*, **1999**,115, 331-334). L'espèce alors marquée par la particule métallique est alors utilisée comme un réactif, qui en combinaison avec des réactifs immunochimiques à base d'anticorps, récepteurs protéinique, haptènes, antigènes, protéines, peptides, oligonucléotides, fragments d'ADN ou ARN, des solutions tampons, d'autres réactifs chimiques, et d'un système de détection électrochimique à base d'électrodes, va permettre d'établir le dosage d'une substance donnée.

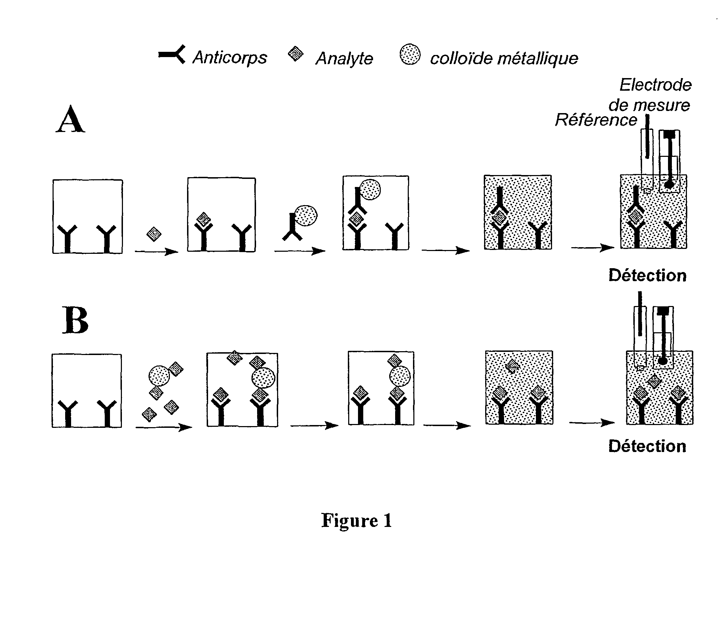

Le principe de l'invention est, à titre d'exemple, illustré Figure 1 dans le cas d'un immunodosage non-compétitif de type sandwich (Figure 1A), ainsi que pour un immunodosage compétitif (Figure 1 B).

Pour la première approche (Figure 1A), le composé à déterminer (l'analyte) est d'abord capturé avec un premier ligand (dans le cas présent un anticorps, pour un test d'hybridation ce serait un oligonucléotide) immobilisé sur une phase solide. La phase solide d'immobilisation du ligand peut être par exemple le fond d'une microcuvette (cas de la Figure 1), la surface d'une microbille (éventuellement magnétique), la surface d'une membrane, ou encore la surface de l'électrode. Après une période d'incubation donnée, éventuellement suivie d'une étape de lavage, un deuxième ligand (ici un anticorps) marqué par un colloïde métallique est ajouté de manière à ce qu'il réagisse avec l'analyte précédemment extrait sur la phase solide. La phase solide ainsi constituée est alors lavée puis traitée par un volume adéquat d'une solution de réactif apte à dissoudre le marqueur métallique colloïdal ayant réagi avec la phase solide. Puis, le métal ainsi solubilisé à l'état d'ions est détecté et quantifié à l'aide d'une électrode soit immergée dans la solution (méthode *in situ -* Figure 1A), soit après transfert de la solution (méthode *ex situ*). La réponse électrochimique peut alors être reliée qualitativement ou quantitativement à la substance à doser.

Dans le cas de la deuxième approche (Figure 1B), la méthode consiste à mettre en contact un échantillon contenant la substance à doser, en présence d'une quantité connue de ladite substance marquée par un colloïde métallique, et d'une certaine quantité de ligand (anticorps) immobilisé sur une phase solide et dirigé contre cette substance. Après un temps de réaction donné, la nature et la quantité de colloïde métallique présent dans la fraction liée est alors, après une éventuelle étape de lavage, déterminée comme indiqué précédemment après dissolution puis détection électrochimique.

L'emploi d'une phase solide constituée par des microbilles, par exemple en latex ou encore en oxyde ferromagnétique, peut être particulièrement avantageux pour améliorer la sensibilité et abaisser la limite de détection. En effet, les microbilles peuvent êtres concentrées après l'étape d'immunoréaction sur une petite surface, comme par exemple la surface d'une membrane filtrante (US 4853335 -Tu *et al., Anal. Chem*. **1993,** 65, 3631-3665) ou encore le fond d'un tube conique, offrant ainsi la possibilité de dissoudre le colloïde métallique dans un volume de liquide inférieur à celui dans lequel a procédé l'immunoréaction.

Les méthodes reposant sur l'agglutination et/ou la précipitation en phase homogène du marqueur métallique colloïdal au cours d'une immunoréaction ou hybridation d'oligonucléotides peuvent également être envisagées (US 5851777). Dans ce cas, les agrégats formés sont isolés puis dissous et détectés comme précédemment.

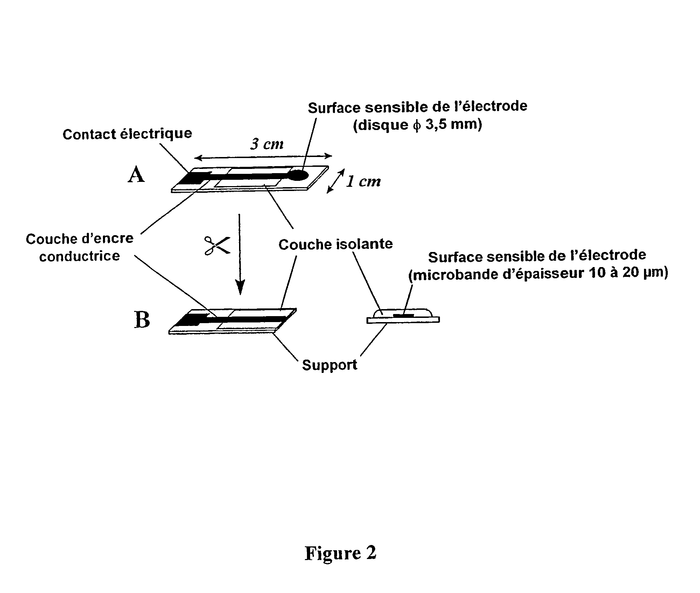

Concernant la nature des électrodes, les électrodes à base de carbone sont préférées, et plus particulièrement les électrodes à disque (Figure 2A) et microélectrodes à bande (Figure 2B) obtenues par sérigraphie d'une encre à base de carbone. Ces électrodes sont en effet particulièrement bien adaptées car elles peuvent être produites en masse à faible coût, et donc être éventuellement à usage unique. De plus, leur forme géométrique ainsi que leur taille peut être aisément modulable. Cependant, d'autres types d'électrodes peuvent être employées telles que des électrodes en carbone vitreux, graphite, matériaux composites contenant du carbone, fibres de carbone, pâte de carbone. D'autre part, la surface de l'électrodes peut être traitée électrochimiquement ou chimiquement afin d'améliorer la sensibilité de détection du métal dissous (Kalcher, *Electroanalysis,* **1990,** 2, 419-433 - Kalcher *et al., Electroanalysis*, **1995,** 7, 5-22 -Ugo et Moretto, *Electroanalysis*, **1995,** 7, 1105-1113). Ce peut être, à titre d'exemple, une modification de la surface de l'électrode ou de la composition de l'encre par un polymère capable d'attirer les ions métalliques *via* une interaction électrostatique ou de complexation, ou encore un prétraitement électrochimique de la surface de l'électrode. Le dépôt d'un film de mercure peut également s'avérer avantageux pour certains ions métalliques difficiles à détecter sur électrode de carbone. Concernant le mode de fabrication des électrodes, la technique de sérigraphie est préférable, bien que d'autres méthodes de fabrication industrielle telles que la rotogravure, l'impression à jet d'encre, éventuellement la photolithographie peuvent être adaptées.

Selon les caractéristiques de l'invention, l'emploi de microélectrodes, de préférence des microélectrodes à bande obtenues par sérigraphie, est particulièrement avantageux car elles offrent la possibilité d'effectuer les mesures dans de très faibles volumes (de l'ordre de quelques microlitres) pour des performances analytiques, en terme de sensibilité et limite de détection en ion métallique, généralement améliorées (Wong et Ewing, *Anal. Chem*. **1990,** 62, 2697-2702 - Wang *et al., J. Electroanal. Chem*., **1993,** 361, 77-83 - Wang et Armalis, *Electroanalysis*, **1995**, 7, 958-961 - Alarnes-Varela et Costa-Garcia *Electroanalysis* **1997**, 9, 1262-1266).

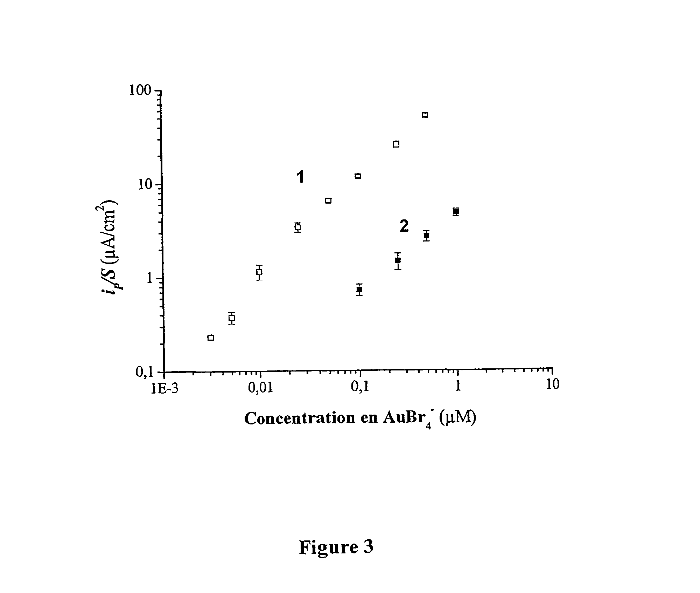

La Figure 3 qui compare les courbes de calibration (densités de courant) de AuBr₄⁻ dans HBr 0,1 M, obtenues sur une microélectrode sérigraphiée à bande de surface S = 1,7 × 10⁻⁴ cm² (courbe 1) et sur une macroélectrode sérigraphiée à disque de surface S = 0,0962 cm² (courbe 2), confirme une meilleure sensibilité dans le cas de la microélectrode à bande. Ces courbes ont été obtenues en voltammétrie linéaire par redissolution anodique de la manière suivante : (i) électrodéposition de l'or à un potentiel constant de E = -0.3 V pendant 300 s, (ii) puis balayage linéaire de potentiel à partir de 0.2 V jusqu'à 1.1 V à une vitesse de 50 mV s⁻¹. Le courant de pic (*i*ₚ) apparaissant vers 1,0 V, lié à l'oxydation de l'or, est pris comme réponse analytique.

Il semble en effet que l'emploi de microélectrodes permette d'obtenir un dépôt du métal qui soit plus efficace qu'avec une macroélectrode. En effet, l'emploi de macroélectrodes nécessite d'agiter la solution, afin d'assurer qu'une quantité suffisante de métal se dépose à la surface de l'électrode. De façon surprenante, les Inventeurs ont observé que l'emploi de microélectrodes permet de s'affranchir de cette étape d'agitation. Ceci pourrait expliquer le gain de sensibilité observé, bien que d'autres hypothèses, en raison de la nature même de la microélectrode (très petite taille), puissent également être envisagées.

Diverses techniques d'analyse électrochimique peuvent être employées pour doser les ions métalliques dissous. Elles sont préférentiellement la voltammétrie (ou polarographie) par redissolution anodique avec un balayage de potentiel pouvant être linéaire, cyclique, à vague carrée, à impulsion normale, à impulsion différentielle, ou à tension sinusoïdale surimposée, ou encore la chronopotentiométrie par redissolution anodique. Cependant, d'autres techniques peuvent être utilisées comme la voltammétrie par échange d'ions, la voltammétrie (ou polarographie) par adsorption et redissolution cathodique avec un balayage pouvant être linéaire, cyclique, à vague carrée, à impulsion normale, à impulsion différentielle, ou à tension sinusoïdale surimposée, ou encore la chronoampérométrie, la chronocoulométrie, la voltammétrie (ou polarographie) linéaire, cyclique, à vague carrée, à impulsion normale, à impulsion différentielle, ou à tension sinusoïdale surimposée. Ces techniques requièrent un montage pouvant être à deux, voire à trois électrodes, c'est-à-dire un montage comprenant l'électrode de mesure précédemment citée, une électrode de référence et éventuellement une électrode auxiliaire. Afin d'éviter une contamination du milieu de dosage par le métal ou l'électrolyte de l'électrode de référence, il est avantageux d'isoler celle-ci par une allonge constituée à son extrémité d'un poreux et remplie d'un électrolyte. Une électrode de référence sérigraphiée à partir d'une encre à base d'argent et de chlorure d'argent peut être également envisagée. Là encore, il peut être utile d'isoler celle-ci par un pont électrolytique, comme par exemple un gel conducteur ionique ou un polymère conducteur ionique, afin d'éviter l'interférence des ions argent au cours d'une mesure.

L'invention concerne également un kit de dosage d'au moins un composé biologique. Selon les caractéristiques de l'invention, ce kit comprend au moins un réactif marqué par une particule métallique colloïdale et au moins une électrode. Le kit selon l'invention peut également contenir au moins un réactif permettant la dissolution de la particule métallique colloïdale, et éventuellement un réactif pour faire disparaître l'excès de réactif d'oxydation. Le kit peut aussi contenir un réactif capable de complexer l'ion métallique, afin de favoriser sa détection. Enfin, le kit peut aussi contenir des instructions afin de permettre la mise en oeuvre du procédé selon la présente invention.

Les exemples suivant illustrent les caractéristiques de l'invention, mais ne doivent pas être considérés comme limitant l'invention.

### DESCRIPTIONS DES FIGURES :

Figure 1. Représentation schématique du principe de l'invention illustrée dans le cas (A) d'un immunodosage non-compétitif et (B) compétitif.
Figure 2. Représentation schématique d'une électrode sérigraphiée (A) à disque et (B) à microbande.
Figure 3. Courbes de calibration de l'or ionique obtenues (1) avec une microélectrode à bande et (2) avec une électrode à disque.
Figure 4. Représentation schématique du dispositif de mesure utilisé pour détecter l'or dissous dans un faible volume d'une goutte de solution.
Figure 5. Courbes de calibration de deux colloïdes d'or recouvert de streptavidine.
Figure 6. (A) Courbe de calibration log-log de l'immunodosage non-compétitif d'IgG. (B) Courbes de voltammétrie par redissolution anodique obtenues pour différentes concentrations en IgG. Les courbes sont repérées par des lettres afin de les faire correspondre aux concentrations indiquées par les mêmes lettres sur la courbe de calibration de l'IgG.

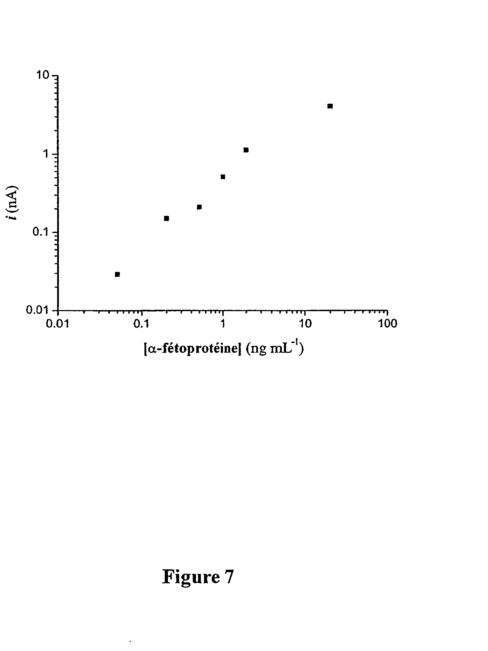

Figure 7. Courbe de calibration log-log de l'immunodosage non-compétitif de l'α-fétoprotéine.

### EXEMPLES

### Exemple 1 : Détection de streptavidine marquée par un colloïde d'or après fixation spécifique au fond d'un micropuits.

Les expériences sont réalisées à température ambiante.

L'albumine de sérum bovine (BSA, fraction V), la biotinamidocaproyle couplée à la BSA (B-BSA, teneur en biotine : 8-12 mol/mol de BSA), la streptavidine marquée avec de l'or colloïdal (S-Au) de diamètre 20 nm, ainsi que la streptavidine couplée à l'albumine sur laquelle sont adsorbées des particules d'or colloïdales de 10 nm (SA-Au), proviennent de Sigma Chemical Co.

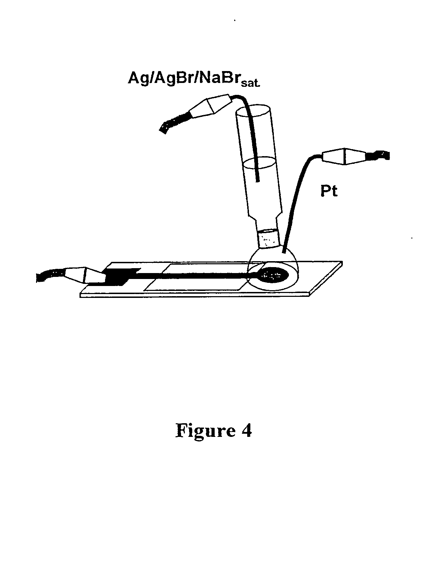

100 µL de B-BSA à 10 µg mL⁻¹ dans un tampon bicarbonate (15 mM Na₂CO₃ ; pH 9,6) sont pipettés au fond d'un micropuits en polystyrène (Nunc) et laissés à incuber pendant 2 heures. Après avoir vidé et rincé le micropuits avec 110 µL de tampon phosphate (PBS : 4,3 mM NaH₂PO₄, 15,1 mM Na₂HPO₄, et 50 mM NaCl ; pH 7,4), 100 µL de PBS contenant 0, 1 % de BSA (PBS-BSA) sont ajoutés et laissés pendant 2 heures à incuber. Le micropuits est ensuite vidé et rincé 3 fois avec 110 µL d'eau pure. Puis 35 µL d'une solution de S-Au ou SA-Au à *x* µg.mL⁻¹ (0,003 < *x* < 3) dans un tampon PBS-BSA contenant 0,05% de Tween 20 (PBS-BSA-T) sont alors introduits dans le micropuits et laissés à réagir pendant 3 heures. Une fois vidé, le micropuits est lavé abondamment avec 3 × 110 µL de PBS-BSA-T, puis avec 2 × 110 µL de PBS. Le colloïde d'or fixé sur les parois du micropuits est alors dissous en introduisant 40 µL d'une solution de Br₂ à une concentration de 10⁻⁴ M dans HBr 1 M. Au bout de 5 minutes, un volume de 35 µL est prélevé du micropuits et transféré sur la surface d'une électrode de carbone sérigraphiée à disque (S = 0,0962 cm², électrode préparée selon la méthode décrite dans la réf. : Bagel *et al., Anal. Chem*. **1997,** 69, 4688-4694), auquel est ajouté 5 µL d'une solution fraîche d'acide 3-phénoxypropionique à 4 × 10⁻³ M dans HBr 1 M. Une électrode de référence (Ag/AgBr, NaBrₛₐₜ) prolongée par une allonge contenant une solution saturée de NaBr, et une électrode auxiliaire sont alors immergées dans les 40 µL de solution précédemment déposés à la surface de l'électrode de carbone sérigraphiée, comme le montre le schéma de la Figure 4. Les mesures de voltammétrie linéaire par redissolution anodique sont alors effectuées en procédant de la manière suivante :
1) électrodéposition de l'or à un potentiel constant de E = -0.3 V pendant 300 s,
2) puis balayage linéaire de potentiel à partir de 0.2 V jusqu'à 1.1 V à une vitesse de 50 mV s⁻¹.

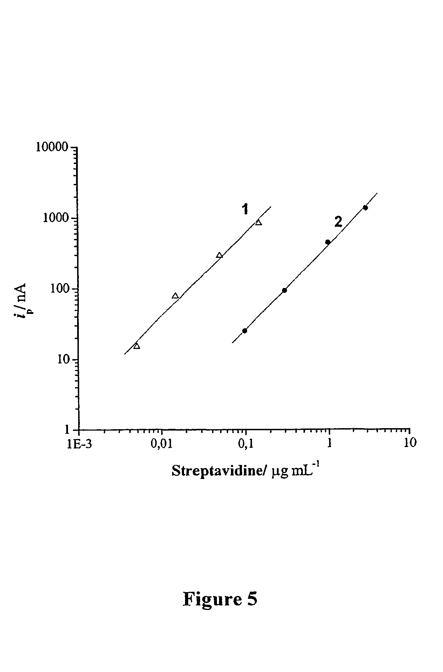

Le courant de pic (*i*ₚ) apparaissant vers 1,0 V, lié à l'oxydation de l'or, est pris comme réponse analytique. La mesure peut également être l'intégrale du pic, ce qui correspond alors à une quantité de coulomb (*Q*ₚ). Les courbes de calibration sont représentées Figure 5 en échelle logarithmique pour chacune des streptavidines marquées à l'or. On peut remarquer une meilleure sensibilité avec la SA-Au (courbe 1) qu'avec la S-Au (courbe 2).

### Exemple 2 : Immunodosage "sandwich" d'une immunoglobuline.

L'ovalbumine (OA, Grade III) et l'immunoglobuline G de chèvre (IgG) est commercialisée par Sigma Chemical Co. L'anti-IgG de chèvre marqué avec un colloïde d'or de 18 nm, ainsi que celui non marqué, sont des anticorps polyclonaux provenant des laboratoires Jackson Immunoresearch.

60 µL d'une solution d'anti-IgG à 24 µg mL⁻¹ dans un tampon PBS sont pipettés dans un micropuits et laissés à incuber pendant 1 heure. Après avoir vidé et rincé le micropuits avec 2 × 100 µL de tampon PBS contenant 0,5% d'ovalbumine et 0,1% de Tween 20 (PBS-OA-T), 100 µL de ce même tampon sont alors ajoutés et laissés à incuber pendant 1 heure. La solution est ensuite aspirée, puis 35 µL d'une solution d'IgG de chèvre à *x* ng mL⁻¹ (0,5 < *x* < 1000) dilués dans un tampon PBS contenant 0,1% de Tween 20, sont introduits et laissés à incuber pendant 40 minutes. Une fois le micropuits vidé et rincé par 2 × 100 µL de PBS-OA-T, 100 µL de PBS-OA-T sont introduits. Après 30 minutes, le liquide est remplacé par 35 µL d'une solution diluée d'anti-IgG marqués à l'or colloïdal (dilution par 45 de la solution commercialisée dans PBS-OA-T), puis mis à incuber pendant 3 heures. Un dernier cycle de rinçage est effectué en lavant 3 fois le micropuits avec 200 µL de PBS-OA-T, suivi de 2 × 200 µL de PBS. Le liquide est alors soigneusement aspiré, puis le colloïde d'or fixé sur les parois du micropuits est alors dissous et détecté comme indiqué dans l'exemple 1.

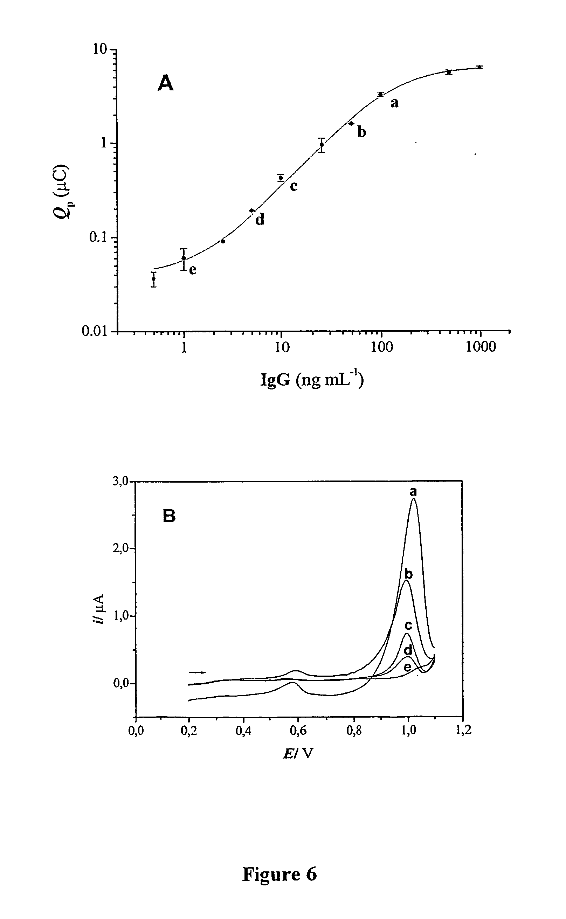

Quelques exemples de mesures obtenues par voltammétrie linéaire par redissolution anodique sont données Figure 6A, tandis que la courbe de calibration de l'IgG de chèvre correspondante est représentée Figure 6B. Chaque point représente la moyenne de 2 mesures et chaque mesure a été obtenue à une électrode différente (électrode à usage unique). Une concentration d'environ 3 × 10⁻¹² M en IgG a pu être déterminée.

### Exemple 3 : Immunodosage non-compétitif de l'α-fétoprotéine humaine.

80 µL d'une solution d'anti-α-fétoprotéine monoclonal (anticorps de souris) à 24 µg mL⁻¹ dans un tampon carbonate (15 mM, pH 9,6) sont pipettés dans un micropuits et laissés à incuber pendant 1 nuit à 4°C. Après avoir vidé et rincé le micropuits avec 2 × 250 µL de tampon PBS-OA-T, 250 µL de ce même tampon sont alors ajoutés et laissés à incuber pendant 40 min. La solution est ensuite aspirée, puis 80 µL d'une solution d'α-fétoprotéine *x* ng mL⁻¹ (0,05 < *x* < 20) dilués dans un tampon PBS contenant 0,1 % de Tween 20, sont introduits et laissés à incuber pendant 2 heures. Une fois le micropuits vidé et rincé par 2 × 250 µL de PBS-OA-T, 250 µL de PBS-OA-T sont introduits. Après 30 minutes, le liquide est remplacé par 80 µL d'une solution diluée d'anti-α-fétoprotéine polyclonal (anticorps de chèvre) dilué à 5 µg mL⁻¹ dans PBS-OA-T et incubé 1 heure. Puis après un rinçage par 2 × 250 µL de tampon PBS-OA-T, 50 µL d'une solution d'anti-IgG de chèvre marqués à l'or colloïdal (dilution par 45 de la solution commercialisée dans PBS-OA-T), puis mis à incuber pendant 1 h 30 min. Un dernier cycle de rinçage est effectué en lavant 3 fois le micropuits avec 250 µL de PBS-OA-T, suivi de 2 × 250 µL de PBS-T, puis 2 × 250 µL de PBS. Le liquide est alors soigneusement aspiré, puis le colloïde d'or fixé sur les parois du micropuits est alors dissous et détecté avec des microélectrodes à bandes de la façon suivante : 50 µL d'une solution de Br₂ 0,1 mM dans 0,1 N HBr sont ajoutés dans les micropuits pendant 30 min ; puis 40 µL sont transvasés dans de nouveaux micropuits contenant 10 µL d'une solution d'acide 3-phénoxypropionique à 2 × 10⁻³ M dans HBr 0,1 M. La détection de l'or dissous est alors effectuée comme indiqué dans l'exemple 1.

### Fabrication de microélectrodes à bande sérigraphiées :

L'encre à base de carbone utilisée pour la fabrication des microélectrodes à bande est une encre commerciale produite par Acheson Colloid (encres Minico® de la série M3000-1RS ou encres Electrodag® telles que 423 SS ou PF 407A). L'encre est sérigraphiée sur un support rigide ou semi-rigide de préférence en polystyrène semicristallin ou en polystyrène choc (plaques d'épaisseur pouvant être comprise entre 0,1 et 2 mm). La finesse du masque de sérigraphie obtenu sur une toile tendue montée sur un cadre, ainsi que la nature, le maillage de la toile, conditionnent en grande partie la qualité du dépôt de l'encre ainsi que son épaisseur. Dans la présente invention des épaisseurs d'encre comprises entre 5 et 50 µm ont pu être obtenues à partir de cadres de sérigraphie comportant 77 ou 120 fils/cm.

Une fois l'encre sérigraphiée, celle-ci est mise à sécher dans une étuve entre 60 et 100°C. Ensuite, une couche isolante à base de polystyrène est déposée ou sérigraphiée de manière à recouvrir une partie de l'encre de carbone précédemment sérigraphiée (Figure 2). Après séchage, l'électrode ainsi constituée est découpée transversalement dans son épaisseur de manière à faire apparaître sur la tranche une bande de carbone d'épaisseur micrométrique (dépendant de l'épaisseur d'encre de carbone initialement sérigraphiée) et de longueur millimétrique (dépendant de la largeur du motif de l'électrode initialement sélectionnée) (Figure 2B).

## Revendications

1. Procédé de détection ou de quantification d'une substance biologique couplée à une particule métallique colloïdale par détection électrochimique, **caractérisé en ce qu'**il comprend les étapes suivantes :
- dissolution par traitement chimique de ladite particule métallique colloïdale dans un milieu acide contenant un réactif oxydant ;
- réduction et/ou précipitation du métal à la surface d'une électrode ;
- mesure de la quantité de métal précipité à la surface de l'électrode par variation du potentiel de ladite électrode et analyse du pic voltamétrique apparaissant après réoxydation dudit métal et sa remise en solution ;
- détection et/ou quantification de la substance biologique couplée initialement à la particule métallique colloïdale, en fonction de la présence et/ou quantité de métal électrodéposé à la surface de l'électrode.

2. Procédé selon la revendication 1, **caractérisé en ce que** l'étape de dissolution de la particule colloïdale métallique est suivie d'un traitement supplémentaire destiné à faire disparaître le produit induisant ladite dissolution.

3. Procédé selon l'une des revendications 1 ou 2, **caractérisé en ce que** l'on rajoute, en solution, un réactif capable de complexer l'ion métallique.

4. Procédé selon l'une des revendications 1 à 3, **caractérisé en ce que** la réduction et/ou précipitation du métal sur l'électrode s'effectue par utilisation d'un potentiel négatif approprié.

5. Procédé selon l'une des revendications 1 à 4, **caractérisé en ce que** la particule colloïdale est choisie parmi les particules constituées de métal, ou de composés métalliques.

6. Procédé selon la revendication 5, **caractérisé en ce que** la particule colloïdale est constituée d'or.

7. Procédé selon l'une des revendications 1 à 6, **caractérisé en ce que** la particule colloïdale présente une taille comprise entre 1 et 200 nm.

8. Procédé selon l'une des revendications 1 à 7, **caractérisé en ce que** le milieu acide contenant un réactif oxydant est une solution d'acide bromhydrique contenant du brome, de l'acide hypobromeux ou un mélange de ces deux composés.

9. Procédé selon l'une des revendications 1 à 8, **caractérisé en ce que** l'électrode utilisée est une électrode sérigraphiée, en particulier une microélectrode à bande.

10. Procédé selon l'une des revendications 1 à 9, **caractérisé en ce que** la surface de l'électrode est traitée électrochimiquement ou chimiquement pour améliorer la sensibilité de détection du métal dissous.

11. Procédé selon l'une des revendications 1 à 10, **caractérisé en ce que** les particules colloïdales présentes en solution après la réaction biologique sont concentrées avant dissolution.

12. Procédé selon l'une des revendications 1 à 11, **caractérisé en ce que** la substance biologique couplée à la particule colloïdale est comprise dans le groupe constitué des anticorps, récepteurs protéiniques, antigènes, haptènes, protéines, peptides, oligonucléotides, fragments d'acides nucléiques.

13. Kit de diagnostic **caractérisé en ce qu'**il comprend au moins un réactif marqué par une particule métallique colloïdale et au moins une électrode, ainsi qu'un reactif oxydant permettant la dissolution de ladite particule métallique colloïdale par voie chimique.

## Patentansprüche

1. Verfahren zum Nachweis oder zur Quantifizierung einer an ein kolloidales metallisches Teilchen gekuppelten biologischen Substanz durch elektrochemischen Nachweis, **dadurch gekennzeichnet, dass** es die folgenden Schritte umfasst:
- Lösen des kolloidalen metallischen Teilchens durch chemische Behandlung in einem sauren Medium, das ein oxidierendes Reagens enthält;
- Reduktion und/oder Fällung des Metalls auf der Oberfläche einer Elektrode;
- Messen der Quantität des auf der Oberfläche der Elektrode gefällten Metalls durch Variation des Potentials der Elektrode und Analyse des voltametrischen Peaks, der nach Reoxidation des Metalls und seiner Wiederauflösung erscheint;
- Nachweis und/oder Quantifizierung der anfänglich an das kolloidale metallische Teilchen gekuppelten biologischen Substanz, als Funktion der Anwesenheit und/oder Quantität von Metall, das auf der Oberfläche der Elektrode elektrochemisch abgeschieden wurde.

2. Verfahren nach Anspruch 1, **dadurch gekennzeichnet, dass** dem Schritt des Lösens des kolloidalen metallischen Teilchens eine ergänzende Behandlung folgt, die dazu bestimmt ist, das Produkt zu beseitigen, das das Lösen herbeiführt.

3. Verfahren nach einem der Ansprüche 1 oder 2, **dadurch gekennzeichnet, dass** man in Lösung ein Reagens dazugibt, welches das Metallion komplexieren kann.

4. Verfahren nach einem der Ansprüche 1 bis 3, **dadurch gekennzeichnet, dass** die Reduktion und/oder Fällung des Metalls auf der Elektrode durch Verwendung eines geeigneten negativen Potentials bewirkt wird.

5. Verfahren nach einem der Ansprüche 1 bis 4, **dadurch gekennzeichnet, dass** das kolloidale Teilchen ausgewählt ist aus den Teilchen, die aus Metall oder metallischen Verbindungen bestehen.

6. Verfahren nach Anspruch 5, **dadurch gekennzeichnet, dass** das kolloidale Teilchen aus Gold besteht.

7. Verfahren nach einem der Ansprüche 1 bis 6, **dadurch gekennzeichnet, dass** das kolloidale Teilchen eine Größe zwischen 1 und 200 nm einschließlich aufweist.

8. Verfahren nach einem der Ansprüche 1 bis 7, **dadurch gekennzeichnet, dass** das saure Medium, welches ein oxidierendes Reagens enthält, eine Lösung von Brom enthaltender Bromwasserstoffsäure, hypobromiger Säure oder eine Mischung dieser zwei Verbindungen ist.

9. Verfahren nach einem der Ansprüche 1 bis 8, **dadurch gekennzeichnet, dass** die verwendete Elektrode eine durch Siebdruck hergestellte Elektrode, insbesondere eine Mikrobandelektrode ist.

10. Verfahren nach einem der Ansprüche 1 bis 9, **dadurch gekennzeichnet, dass** die Oberfläche der Elektrode elektrochemisch oder chemisch behandelt ist, um die Empfindlichkeit des Nachweises des gelösten Metalls zu verbessern.

11. Verfahren nach einem der Ansprüche 1 bis 10, **dadurch gekennzeichnet, dass** die in Lösung vorhandenen kolloidalen Teilchen nach der biologischen Reaktion vor dem Lösen konzentriert werden.

12. Verfahren nach einem der Ansprüche 1 bis 11, **dadurch gekennzeichnet, dass** die an das kolloidale Teilchen gekuppelte biologische Substanz in der Gruppe eingeschlossen ist, die aus Antikörpern, Protein-Rezeptoren, Antigenen, Haptenen, Proteinen, Peptiden, Oligonukleotiden, Nukleinsäure-Fragmenten besteht.

13. Diagnosekit, **dadurch gekennzeichnet, dass** es mindestens ein mit einem kolloidalen metallischen Teilchen markiertes Reagens und mindestens eine Elektrode sowie ein oxidierendes Reagens umfasst, welches das Lösen des kolloidalen metallischen Teilchens auf chemischem Weg ermöglicht.

## Claims

1. Method for detection or' quantification of a biological substance coupled to a colloidal metal particle, by electrochemical detection, **characterized in that** it comprises the following steps:
- dissolving said colloidal metal particle by chemical treatment in an acid medium containing an oxidizing reagent;
- reducing and/or precipitating the metal at the surface of an electrode;
- measuring the quantity of metal precipitated at the surface of the electrode by variation of the potential of said electrode and analysis of the voltammetric peak which appears after reoxidation of said metal and redissolving thereof;
- detecting and/or quantifying the biological substance initially coupled to the colloidal metal particle, as a function of the presence and/or quantity of metal electrodeposited at the surface of the electrode.

2. Method according to Claim 1, **characterized in that** the step of dissolving the colloidal metal particle is followed by an additional treatment intended to eliminate the product inducing said dissolution.

3. Method according to either of Claims 1 and 2, **characterized in that** a reagent capable of complexing the metal ion is added, in solution.

4. Method according to one of Claims 1 to 3, **characterized in that** the reduction and/or precipitation of the metal on the electrode is carried out using a suitable negative potential.

5. Method according to one of Claims 1 to 4, **characterized in that** the colloidal particle is chosen from particles consisting of metal or of metal compounds.

6. Method according to Claim 5, **characterized in that** the colloidal particle consists of gold.

7. Method according to one of Claims 1 to 6, **characterized in that** the colloidal particle is between 1 and 200 nm in size.

8. Method according to one of Claims 1 to 7, **characterized in that** the acid medium containing an oxidizing reagent is a solution of hydrobromic acid containing bromine, hypobromous acid or a mixture of these two compounds.

9. Method according to one of Claims 1 to 8, **characterized in that** the electrode used is a screen-printed electrode, in particular a strip microelectrode.

10. Method according to one of Claims 1 to 9, **characterized in that** the surface of the electrode is treated electrochemically or chemically in order to improve the sensitivity of detection of the dissolved metal.

11. Method according to one of Claims 1 to 10, **characterized in that** the colloidal particles present in solution after the biological reaction are concentrated before dissolution.

12. Method according to one of Claims 1 to 11, **characterized in that** the biological substance coupled to the colloidal particle is comprised in the group consisting of antibodies, protein receptors, antigens, haptens, proteins, peptides, oligonucleotides, and nucleic acid fragments.

13. Diagnostic kit, **characterized in that** it comprises at least one reagent labelled with a colloidal metal particle and at least one electrode, and also an oxidizing reagent allowing the chemical dissolution of said colloidal metal particle.
